# EUROPEAN PATENT APPLICATION

(11) **EP 4 272 650 A1**
(43) Date of publication of application: **08.11.2023**
(21) Application number: 22172082.4
(22) Date of filing: 06.05.2022
(51) Int. Cl.: A61B 6/02, A61B 5/103, A61B 5/00, A61B 6/04

(54) **OPTICAL FIBER BASED 3D POSITIONING AND TRACKING OF PATIENT BODY PART DURING X-RAY**

(71) Applicant: Koninklijke Philips N.V., 5656 AG Eindhoven (NL)
(72) Inventor: CHAUDHURY, Sudipta, Eindhoven (NL); CHAKRABARTI, Biswaroop, Eindhoven (NL); PAUL, Soubhik, Eindhoven (NL); KRÖNKE, Sven, Eindhoven (NL); VON BERG, Jens, Eindhoven (NL); BRUECK, Heiner, Matthias, Eindhoven (NL); YOUNG, Stewart, Matthew, Eindhoven (NL); WIEBERNEIT, Nataly, Eindhoven (NL)
(74) Representative: Philips Intellectual Property & Standards

(57) **Abstract**

A system (SYS) and related method for facilitating medical imaging of a patient by a medical imaging apparatus (IA). The system comprises an input interface (IN) for receiving measurements collected by shape sensing sensors (Sⱼ) of an optical shape sensing device (SSD). The shape sensing sensors (Sⱼ) are arrangeable relative to the patient's body, wherein the measurements are representative of a current posture of the patient's body. A posture determiner (PD) of the system (SYS) computes, based on the measurements, output data representative of whether the patient's body is in a predefined target posture.

## Description

### FIELD OF THE INVENTION

The invention relates to a system for facilitating medical imaging of a patient by a medical imaging apparatus, to an imaging arrangement using such as system, to a related method, to a computer program element, and to a computer readable medium.

### BACKGROUND OF THE INVENTION

Chest x-ray is one of the most established first line imaging modality for investigating abnormalities in the thorax region for example, or for orthopedic (e.g., limbs) or dental use cases and others.

Patient position and patient rotation relative to the X-ray source and in particular the X-ray detector are two important quality aspects, that have major implications on chest radiograph interpretation and diagnosis for disease.

In present hospital or diagnostic center workflows, error pertaining to patient position and orientation (referred to collectively herein as patient pose or posture) can be detected before (e.g., by using a camera) or after acquiring the imagery (radiograph), for example by analyzing various image features. Feedback can be provided to correct for such posture error.

Once the patient posture error is detected by whichever means, a next step is to correct the patient posture and perform imaging.

In case of chest radiographs, an absence of rotational asymmetry, i.e., ensuring that the straight line formed by the spinous processes is equidistant from the medial ends of the clavicles or the scapulae is important for biometry in the radiograph and in avoiding false inference. This may call for a specific posture in relation to patient's torso. There may also be another specific posture required in relation to patent's arms in order to rotate the scapulae outside the lung field of view. Correct posture is even more important for limb radiographs where wrong positioning for example adversely affects interpretation.

It is not uncommon that several acquisitions are required until a diagnostic musculoskeletal image is achieved.

For radiation protection and to save time it is desirable to be aware of any such posture errors, and to correct posture before imaging to "get it right first time".

### SUMMARY OF THE INVENTION

There may be a need for an efficient manner of imaging, in particular in X-ray projection imaging, or in other modalities, X-ray or not.

An object of the present invention is achieved by the subject matter of the independent claims where further embodiments are incorporated in the dependent claims. It should be noted that the following described aspect of the invention equally applies to the related method, to the imaging arrangement, to the computer program element and to the computer readable medium.

According to a first aspect of the invention there is provided a system for facilitating medical imaging of a patient by a medical imaging apparatus, comprising:
an input interface for receiving measurements collected by shape sensing sensors of an optical shape sensing device, the shape sensing sensors arrangeable relative to the patient's body, wherein the measurements are representative of a current posture of the patient's body; and
a posture determiner configured to compute, based on the measurements, output data representative of whether the patient's body is in a predefined target posture.

The set of sensors may be arranged along a length, such as of a fiber, wire or other elongated structure. Preferably, optical shape sensing is used. Preferably, the elongate structure is deformable to respond to posture changes, in turn causing shape sensor readings or measurements that represent posture changes. The set of sensors (sensor elements) may include cores of optical fibers and/or gratings (such as Fiber Bragg gratings) or other arrangements included in a given optical fiber core, capable of measuring shape changes. The set of sensors are so arranged on the patient that relevant posture change causes a change of shape of elongated structure which is captured by the set of shape sensing sensors.

The target posture may include in particular "local" posture, that is a spatial mutual configuration of anatomies and or body parts of a given patient. "global" posture includes relating "local" posture to an external coordinate system such as the detector or other object, such as X-ray source, etc. Posture may relate to posture of arm(s), leg(s), torso, head, etc, but may also include posture such as inhalation status as this relates to a certain posture of the chest.

In embodiments, the system comprises an output interface for providing the output data, the output data including and one or more of i) an indication for the said current posture, ii) an indication on when the patient's body is determined to be in the predefined target posture, and iii) indication on when there is a deviation between the said predefined target posture and the current target posture.

In embodiments, the output interface includes any or more of: a display device, a haptic actuator. For example, the haptic actuator may be integrated in, or attached to, the sensors. In general, the output interface is configured to support any or one or more of a range of imaging tasks, including controlling operation of the imaging apparatus. Thus, the output interface may include one or more suitable control interfaces. The output interface may be used to provide all manners of preferably real-time feedback to user on patient posture and/or whether this posture is the target posture.

In embodiments, the system comprises a logic configured to recommend to a user to initiate imaging, or the logic to automatically initiate the imaging, if the output data is indicative of the patient's body being determined to be in the predefined target posture. The logic may prevent imaging until the output data is indicative of the patient's body being determined to be in the predefined target posture. The logic may use the control interface for the control task.

In embodiments, the measurements include measurements collected by further set of shape sensing sensors arrangeable in a predefined spatial relationship to a detector module of the medica imaging apparatus.

In embodiments, the further set of shape sensing sensors are arrangeable in a layout that defines a reference plane that may serve as frame of reference to determine patient posture relative to the detector.

In embodiments, the said further shape sensing sensors are arrangeable in, at or on the detector module.

In embodiments, the shape sensing sensors are arrangeable on the patient's body.

In embodiments, the shape sensing sensors are arrangeable at anchor points that define a region of interest.

In embodiments, the shape sensing sensors are includable or couplable in or to a wearable, such as garment.

In embodiments, the measurements include yet further measurements collected by yet further shape sensing sensors arrangeable at the imaging apparatus, wherein the system further includes an imaging geometry determiner, configured to determined based on the further measurements, a current imaging geometry of the imaging apparatus.

For example, the said imaging geometry includes any one or more of SID (source-detector distance), inclination of detector plane relative to an imaging axis of imager, running from center point of detector plane to focal spot of imager's source. The logic may thus take such imaging geometry into account and allows imaging only if the correct imaging geometry is assumed. Thus, the system may monitor for patient posture and imaging geometry. Doing both in combination is preferred, but each may be done independently without the other in some cases.

In another aspect there is provided an imaging arrangement, including a medical imaging apparatus, and shape sensing sensors arranged at the imaging apparatus, and supplying the shape measurements to a system for determining patient posture.

In embodiments, the imaging apparatus has a detector module, the shape sensing sensors arranged on or at the detector module. This arrangement allows in particular to define a (reference) plane/frame on/in relation to the said detector module and allow determining robustly and accurately patient posture relative to the detector for better image quality.

In yet another aspect there is provided a wearable including such a set of sensors, such an optical fiber, and a transmitter interface for transmitting the readings to a system for processing the readings into posture changes in relation to a patient wearing the wearable.

In another aspect there is provided a computer-implemented method for facilitating medical imaging of a patient by a medical imaging apparatus, comprising:
receiving measurements collected by shape sensing sensors of an optical shape sensing device, the shape sensing sensors arrangeable relative to the patient's body, wherein the measurements are representative of a current posture of the patient's body; and
computing, based on the measurements, output data representative of whether the patient's body is in a predefined target posture.

The method may further include establishing, based on the output data, whether the patient's body is in a predefined target posture.

The method may further include performing a control task to support imaging, based on the established outcome.

A reference to the "*patient's body*" above may relate to the whole body or merely a part or parts thereof.

In yet another aspect there is provided a computer program element, which, when being executed by at least one processing unit, is adapted to cause the processing unit to perform the method.

In yet another aspect there is provided at least one computer readable medium having stored thereon the program element.

In yet another aspect there is provided a wearable including an optical fiber and a transmitter interface for transmitting the readings to a system for processing the readings into posture changes in relation to a patient wearing the wearable.

In embodiments, the one or more sets of sensors are, or include, a respective optical fiber. The one or more fibers are used to collect data on posture of the relevant anatomical region(s) of the patient's body (curvature points), and, in some embodiments, of a reference frame in relation to the detector module of the imaging apparatus. One set of sensors is at the patient and another set of sensors are at the X-ray detector of the imaging apparatus. A deviation of current patient posture from a target posture can be so measured. Preferably, real-time feedback on posture deviation for posture correction can be provided by driving a display device, controlling a lamp, an acoustic transducer, a haptic transducer, or any other suitable transducer. The feedback may be provided to user and/or patient.

In more detail, and in some embodiments, the system may include a set of such sensors as optical fibers (such as strips, or other elongated form). One or more of the said fibers may be attached to patient body with respect to the anatomical region which is to be imaged. This optical fiber ("patient fiber"), is used for curvature sensing caused by patient movement. Another optical fiber may be arranged on a circumference of the detector module. This fiber provides a reference frame for the curvature sensing captured through patient fiber. Preferably, both fibers are attached to the same sensing system. Preferably, both are suppled from the same light source (transmitter) of the shape sensing system. A LASER based shape sensing system may be used. Use of the same light source allows defining a common coordinate system and allows measuring relative distances between any pair of points on the two fibers. For example, in a chest radiograph, one optical fiber is attached to the detector module, and is arranged along the detector module's circumference. The patient fiber may be attached to patient, and may be run for example, from the wrist of one hand to the wrist of other hand passing though the shoulder area. Many other such arrangement layouts on the patient are envisaged in embodiments. For example, patient fiber(s) may be stuck to patient's skin, or may be embedded within clothing items (socks, shirts, headbands, etc) or may be attached or otherwise affixed to patient by using elasticated attachment systems, and the like. Other layouts may be required for imaging tasks other than for chest imaging. However, even in chest imaging, other fiber layouts than via wrists and back are also possible and envisaged herein.

The system and method described allow for sufficiently accurate localization of the patient's body part in 3D space, with respect to the detector for example, which in turn facilitates posture correction. However, use of the second set of shape sensors at the detector is not necessarily required herein in all embodiments, and the shape sensing measurements collected at patient alone may be sufficient, for example with suitable prior calibration. Use of as the second set of sensors at the detector is preferred herein as this allows more robustly and accurately determined correct patient postured with respect to the detector (and optionally the X-ray source). It may also be possible to place the (one or more) second set of sensors at the X-ray source instead of ta the detector. In another embodiment, at least two additional sensor sets may be used, one at the detector and one at the X-ray source.

The system and method proposed herein allow sufficiently precise determining of patient posture (in relation one or more body parts), prior to and during imaging (such as in radiography), with easy integration in current standard of care workflow.

Optionally, patient's posture relative to X-ray detector can be determined by using in addition to the patient fiber the second set of sensors/second fiber. Such a system is more robust against changes to imaging geometry, such as posture changes of the detector (re-orientation or repositioning).

Optional, real-time tracking of posture may be done by implementing the system on performant computing equipment.

Ambiguity during interpretation regarding the image appearance can be reduced or avoided, thanks to correct posture, which in turn results in correct findings. The likelihood for retakes can be reduced, saving dose and time, which is welcome by users and patients alike.

The proposed system to superior to previous camera-based system. Any cameras can be avoided altogether, leading to better patient privacy. No sensitive data need be collected. Also, camera-based posture control (such as RGB or depth cameras) are expensive, require complex calibration and setup overhead, and their field of view tends to be limited, especially by occlusion. That being said, the proposed system may be used, if required, alongside or in combination with such camera-based systems.

The principles proposed herein are applicable to re-configurable setups (such as mobile x-ray) or to fixed settings.
*user*" relates to a person, such as medical personnel or other, operating the imaging apparatus or overseeing the imaging procedure. In other words, the user is in general not the patient.
"*patient*" is the object of the imaging. Reference to "patient" is not necessarily a reference to patient as whole, but may be a reference to a part, such as body part, anatomical feature and the like. Thus, an object of the imaging may relate to such a part, referred to herein as the region of interest (ROI). Patient as used herein mainly for human patients, but animals, such as pets, in veterinary applications, are not excluded herein.
*"posture* /*pose*" is used interchangeably herein and relates to position and orientation of a body part, organs, etc, or of anatomical feature more generally, relative to a coordinate frame, local or global. Posture may include multiple different postures, one for each body region, which may or may not include the ROI itself. Posture/pose particular may include "local" posture, that is a spatial mutual configuration of anatomies and or body parts of a given patient. "global" posture includes relating "local" posture to an external coordinate system such as the detector or other object.

### BRIEF DESCRIPTION OF THE DRAWINGS

Exemplary embodiments of the invention will now be described with reference to the following drawings, which, unless stated otherwise, are not to scale, wherein:
Figure 1 shows a block diagram of an imaging arrangement including an imaging apparatus;
Figures 2a-c show a block diagram of a shape sensing device in various embodiments as may be used in arrangement of Figure 1;
Figures 3a, b show shape sensing sensors arranged at a patient, a detector, or integrated in a wearable;
Figure 4 shows a block diagram of a computerized system for assisting in patient posture tracking or facilitating patient posture control, based on shape measurements receivable from a shape sensing device;
Figure 5 illustrates processing of shape sensing data;
Figure 6 illustrates use of shape sensing sensors in an orthopedic application; and
Figure 7 shows a flow chart of a computer implemented method for patient posture tracking or facilitating patient posture control, based on shape measurements receivable from a shape sensing device.

### DETAILED DESCRIPTION OF EMBODIMENTS

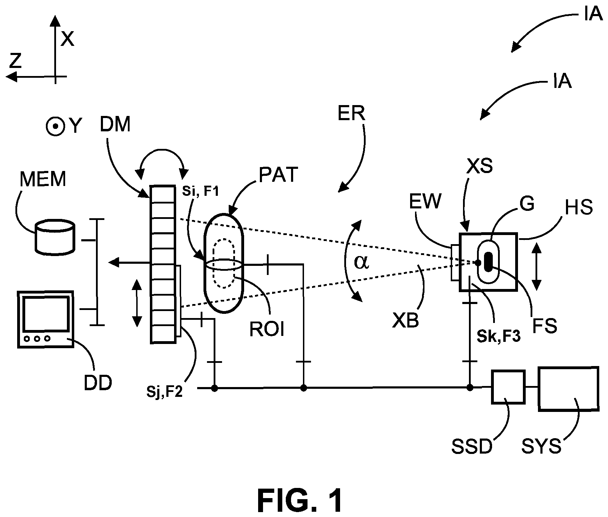

Referring first to the block diagram in Figure 1, this shows components of a medical imaging arrangement AR envisaged herein in embodiments.

The arrangement AR preferably includes an imaging apparatus IA, such as an X-ray based imaging apparatus, a radiographic imager, a C-arm, or the like of the mobile or fixed type. Projection imaging is mainly envisaged herein, but tomographic reconstructions such via as a CT scanner are not excluded herein. The imager IA is operable to acquire medical imagery of a patient PAT during an imaging session to aid, for example, therapy and/or diagnosis. An example of this is chest X-ray imaging as schematically shown in Figure 1, but imaging for other body parts or other purposes are also envisaged herein. Optical imaging, as an alternative or as an addition to X-ray imaging is not excluded herein, such as for dermatological exams.

The imaging arrangement AI includes a computing system SYS implemented by one or more fixed or mobile computing devices. Broadly, the computing system SYS provides computer-assisted facilitates patient posture control and/or tracking, preferably in 3D, during or before the imaging session. Posture may relate to a relevant body part or parts of the patient. The relevant body part may include the region of interest ROI, for example, one or more lungs of the patient. The region of interest may include features internal to the patient's body, such as internal anatomies, organs, tissue and the like, but relevant body parts may also include external features such as on extremities, torso, head etc. Facilitating patient body posture correction and/or tracking is beneficial for accurate imaging. It is preferable to ensure that the region of interest is within the field of view (FOV) of the imager IA. However, this is not always sufficient for good image quality. In addition, what is also ensured herein thanks to the system SYS is that the ROI is in the FOV at a desired target body posture. Posture may relate to position and/or orientation of the ROI but also to position and/or orientation of the body part or parts that include the ROI. In particular, correct posture of the ROI is preferably in conjunction with a correct posture of anatomic features (anatomy, body parts, tissues, etc) that surround or neighbor the ROI. As a specific example, in chest-X-ray, an arm posture may be preferred, so that the shoulder blades are moved outside the field-of-view for better lung tissue imaging.

The FOV is defined by an x-ray beam XB generated by an x-ray source XS of the imaging apparatus IA ("imager"). Specifically, an x-ray generator G, such as an X-ray tube inside a housing HS of the x-ray source, causes, upon electrical energization, the beam XB to issue forth from a focal spot FS of the generator G, to egress the housing HS through an egress window EW, and to propagate along an optical axis through open space traversing examination region. Examination region ER includes the portion of space defined between an x-ray detector DM and the said x-ray source XS of imager IA.

During imaging, patient PAT resides in examination region ER as shown in Figure 1. Patient PAT may squat, lie, sit, stand or otherwise reside in examination region ER. For example, patent PAT may stand upright in the examination region as is done according to some protocols in chest X-ray imaging as sketched in Figure 1. The x-ray beam XB traverses the examination region and patient tissue situated therein. The x-ray beam interacts with patient tissue, is then modified due to the interaction, and it is the so modified x-ray beam that is detected by detector pixels of the module DM. The detector pixels are mounted inside housing H of the detector module DM. The said x-ray sensitive detector pixels are arranged preferably in 2D, such as in a matrix layout. The x-ray sensitive pixels detect the impinging x-radiation in form of intensities. An A/D conversion circuitry of detector module DM converts intensities into digital imagery, for example projection imagery.

The imagery may aid for example diagnosis by revealing inner structures of the lung for example to help diagnose certain lung conditions. The imagery may be stored in an image database MEM, and/or may be processed by a visualizer VIZ to produce a visualization for displaying on a display device DD. The imagery may be mapped onto a suitable grey value or color palette which is used to drive video circuitry which in turn causes the display device to display the visualization. The imagery may be otherwise processed in addition or instead. Attenuation based projection imaging is mainly envisaged herein, however, other modalities such as phase contrast and/or dark field imaging are not excluded herein.

The example imaging set-up as shown in Figure 1 envisages an imaging apparatus IA of the free type in which there is no or no permanent mechanical connection between the x-ray source and the detector module DM. Detector module DM and/or the x-ray source XS are preferably each so arranged that imaging geometry may be changed. For example, this may include modifying position or orientation of detector DM and/or source XS. Position and/or orientation be changed independently. It may be changed automatically or manually by the user in correspondence to the protocol and imaging task at hand. For example, x-ray source and/or detector module may each be subjected to independent any one or more of yaw, pitch and roll movements as indicated in Figure 1. Examples of those degrees of freedom in relation to imaging geometry are illustrated by curved or straight double-arrows in Figure 1. For example, an orientation may be changed, such as direction of the imaging axis along which the beam XB propagates. In addition or instead, a direction of a normal vector of the detector plane may be changed. In addition or instead still, source XS and/or detector DM may experience translation. The mutual spatial configuration, defined by the respective pose of source XS and detector DM may be referred to herein as an imaging geometry. Imaging geometry may further include source-detector distance which may be changed, as required.

The imaging geometry changes of x-ray source and detector module may be defined with reference to a global co-ordinate system (*X,Y,Z*) as indicated in Figure 1. Directions (*X,Y*) perpendicular to each other for example, indicate in general the image plane as defined by the x-ray sensitive pixel layout of the detector module DM. Y direction extends into the drawing plane of Figure 1. Direction *Z* indicates in general the imaging axis along which broadly the x-ray beam propagates through space. The inclination or pitch of the x-ray beam can be changed in response to suitable pitch movement of the x-ray source as indicated by vector α. Similar such degrees of freedom (DOFs), or more restricted ones, are not confined to imagers of the free type however, but may also be afforded by imagers with mechanically coupled setups, such in a C-arm apparatus or by a radiography apparatus, etc.

The mentioned (optional but preferable) multiple DOFs of the detector module DM and/or of the x-ray source XS allow precise adjustments of imaging geometry for the imaging task at hand to maximize imaging value. It can thus be ensured that the region of interest is optimally exposed to x-ray radiation. Intervening body parts, structures or anatomies are kept at a defined relationship, either inside or outside of the FOV, as required. In particular in projection imagery, intervening anatomy may lead to overlapping imaging structures, thus occluding potentially vital image information. This may be undesirable in particular in diagnostic tasks. For example, there may be a desired to keep intervening structures, features outside of the FOV as defined by the x-ray beam by asking the patient to assume the desired target postured specific to the imaging task at hand. However, such correct posture control, especially in 3D as shown in Figure 1, may be challenging as it requires good co-operation of the patient. Patient is expected to assume pre-defined target body posture and maintain this during the imaging session whilst he or she is exposed to radiation XB. Incorrect body posture may render the acquired imagery undiagnostic, in other words, useless. Image-retakes may be required which consumes extra time and/or incurs additional dosage cost for patient and/or staff.

The computerized system SYS is thus configured herein to assist in body posture control or tracking during imaging so as to ensure the target body posture is maintained preferably at all times during the imaging. In particular and preferably, imaging may start only once the target position has been assumed by patient. As will be explained in more detail below the system uses measurements received by a shape sensing device SSD.

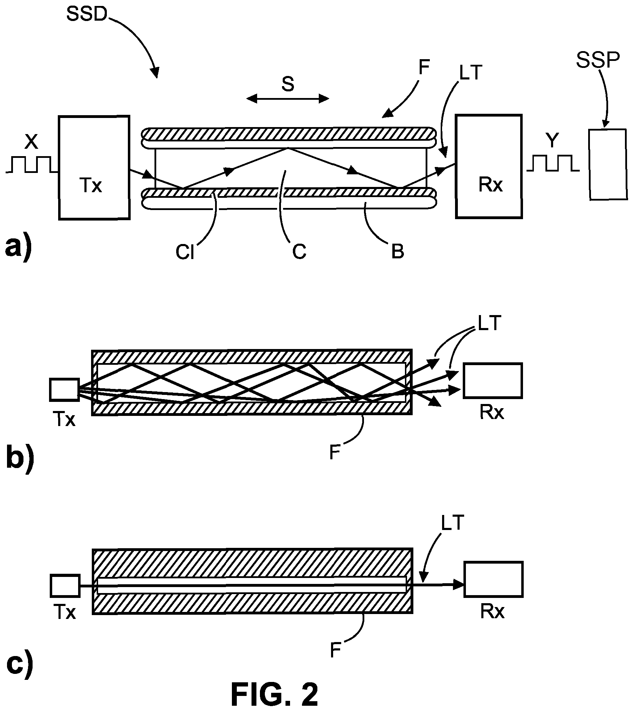

The measurements may include shape sensing measurements. The measurements are processed by system SYS to produce for example an indication of the correct imaging target posture of the patient. Suitable such shaping sensing measurement devices SSD are preferably of the multi-channel type. Examples are shown schematically in the block diagrams of Figures 2a)-c).

The shape sensing device SSD is one embodiment of a posture measurement device configured to measure, using sensors to be described in more detail below, a current patient posture. The measured current patient posture is preferably measured relative to the detector module DM. In addition or instead, the shape sensing device SSD measures whether there is deviation from the intended target posture envisaged for the imaging task at hand. The current measured patient posture or deviation may be provided by the device SSD as an output result indicative.

Respective different pre-defined target postures may be held in a memory and may be accessed by the system once user specifies on a user interface the intended imaging task type/protocol etc. The output result is indicative of the said patient posture, either on its own (with reference to a global coordinate frame), or, preferably, with reference to the detector module, in particular to a local coordinate detector coordinate frame associated with the detector module DM.

The computed result may be used to facilitate good image quality (IQ), as the feed of shapes sensing readings allows dynamic and preferably real-time monitoring of patent posture. The result may also be used to facilitate posture control or correction, such as by providing guidance through suitable transducers, such as acoustical, optical, haptical, or other.

Broadly and preferably, optical shape sensing is preferred herein, but other non-invasive and non-nonionizing shape sensing principles and technologies are not excluded herein. The shape sensing device SSD includes a shape sensing processing unit SSP and plural sensors S, mentioned briefly above. The sensors S are preferably defined in or on, but in either case along, a deformable elongated component of the sensor device, such as a probe, arm or the like. The shape sensing device is preferably based on optical light in which case the said probe or arm may be implemented as a length of an optical fiber F. Preferably, multiple such fibers may be used that each define a separate set of sensors. Preferably at least two, more than two or all fibers are coupled to the same shape sensing processing unit SSP, and their shape sensing readings are processed together to produce the output result. The fiber F may have circular, elliptic or other such cross-section, or may be instead arranged flat, as a band, strip, or tape for example. Suitable such optical fibers are described in Applicant's US 2009/0137952.

One way posture may be measured is by arranging a first set sensors Si (also referred to herein as "patient sensors") of the shape measurement device SSD in relation to the patient, in particular in spatial relation to the region of interest.

Optionally, a second set of such sensor Sj (referred to herein also as "auxiliary sensors") is arranged in spatial relation to the detector module DM. The auxiliary sensors in respect of detector module may be arranged at or on the mobile DM or may be integrated therein. However, it may be sufficient for the sensors to be arranged in a prior known spatial relationship, not necessarily at the detector, but elsewhere in the examination room or at the other parts of the x-ray imager IA, arrangement of the second set of sensors Sj. In the following, a reference to the/a "*fiber F*" may be taken in addition as a reference to the respective set of sensors that makes up at least a part of that fiber F.

For example, (first) fiber F,F1 (and hence the first set of sensors Si) may be mechanically coupled to the patient at or around the region of interest. In particular, the first set of sensors Si/fiber F1 may be coupled to that part of the patient's body which includes at least partly the ROI, but may be arranged to extend beyond said body part(s). Generally, the sensors/fiber are/is arranged spatial in manner so that the sensos are capable of respond with different shape sensing readings that vary with changes to the relevant posture. Thus, preferably any motion of the relevant body part, or relevant posture change in general, is thus imparted onto the fiber F1, which deforms in response to the patient posture change, thanks to the mechanical coupling. The motion may be local or global. Local motion should have preferably direct effect on certain prechosen anchor-points (to be described below in more detail) through which the fiber runs to impart the motion information. Certain global motion may also affect the said fiber anchor points. It is motion with respect to the field of view that is of main concern herein.

This deformation causes light that travels through the optical fiber to be internally reflected in a particular reflection pattern that correlates with deformation of the fiber F. Thus, the reflection pattern can be correlated to curvature that the fiber is experiencing along its length. As each sensor or point along the length of fiber experience its own location curvature, a corresponding set of curvature values (one for each senor) may be provided as output.

The shape sensing processor may compute from the curvature values, 2D or preferably 3D point coordinates as the output result. However, such conversion is not necessarily required, and output may be provided as curvature data instead. in fact, the format or nature of the output data is immaterial herein, so long as the output data is indicative to the said patient posture and/or target posture deviation. The output data may relate to stress or strain measurements. Basic operation of the above-described optical shape is illustrated in block diagram of Figure 2a).

An input signal X controls a light transmitter TX to transmit light through fiber F. A cross section of core of such an optical fiber is shown, and it will be understood that there may be more than one such cores wound, braided or bundled to otherwise combined so make up the fiber. A cross section of the fiber is shown, with a core C and optional cladding zone buffers in which the core is ensheathed. Light ray travelling through fiber F undergoes (internal) total reflections and is received at light detector RX to produce an output measurement signal that represents the reflection pattern experienced by the ray and correlatable with shape of the fiber, and hence body posture due to attachment of fiber to relevant body part as described above. The reflection patten may be processed by processor SSP into the said indicative output signal. The processor SSP may include a solver to solve, based on the shape measurements (stress, strain or curvature values), a set of Frenet-Serret equations to obtained output data indicative of shape and/or 3D positions that represent posture.

Similar readings may be received from optional second fiber F2 (for example coupled to detector module) to provide a second stream of shape sensing readings which can be co-processed with stream of shape readings from the first fiber F1, to more robustly compute posture relative to detector module DM. Using a single or more fibers arranged solely at the patient may also be possible, in which case no fiber(s) F2 need be arranged in relation to the detector module DM.

It will be understood that in some embodiments, the sensors Si,j may be discretely arranged as a set of Fiber-Bragg-Grating (FBG) sensors, although this is not necessarily required in all embodiments as other technologies are also envisaged. For example, the sensors may include point positions or locations along the fiber, so are not necessarily discrete components/structures as in FBG or similar, but are rather quasi-continuous lineal arrangements of point locations along core C of the fiber.

In other arrangements, the FBG per core are not necessarily spread out evenly over the core's length, but are arranged instead towards an end portion of the respective core, or elsewhere along the respective length of the respective core. There are preferably plural fiber cores per fiber F, such as 2 or 3 or more. 3 cores is sufficient for good results in 3D, but 2 may suffice in some circumstances. Thus, a reference herein to a set of sensors S, may be construed as a reference to the respective multiple cores of the fiber F and/or to multiple FBGs in a core, or to other arrangements in or at the fiber core(s) that enable measuring shape.

A reference sign "S" is a generic reference to such as set of shape sensing sensor(s), and a reference sign "F" is as generic reference to such an optical fiber, no matter where the set S/fiber F is located. A reference to fiber is in general a collective reference to the core(s) of which the fiber is made up.

Specifically, light transmitted through an optical fiber may be used to estimate the 3D curvature of the plane where the optical fiber is placed. In this process, different wavelengths of light are transmitted through the optical fiber and the phase difference and Time of Arrival (TOA) is calculated. From these phase differences and TOAs, approximation of the curvature points of the plane is derived. Alternatively, the mentioned FBG-based sensors are used. Each point on the curvature may be defined by a tuple (*x, y, z*), representing the positional vector in the 3D space. Sending the different wavelengths may be done sequentially in single mode (Figure 2c) where a single light ray at a given wavelength is transmitted at a time, or by using multi-mode fiber optics (Figure 2b) where lights at different wavelengths are send down the fiber F at the same time.

In general, in the sensing operation, points of references may include inflection points on the fiber core. The shape sensing mechanism in general monitors the respective angle formed at the inflection points. If a second set of sensors at the detector is used. as is preferred herein, alignment of the angles with the reference frame is monitored. The reference frame is defined by the second set of sensor Sj at the detector, in addition to the set of sensors Si at the patient.

It will be understood that the embodiments of the shape sensing device SSD in Figure 2a-c are merely examples and other arrangements are also envisaged herein so long as shape measurements are obtainable that are correlatable to posture changes, substantially as described herein. Optical light or infrared light is used herein, but other frequencies in the non-ionizing range are also envisaged herein.

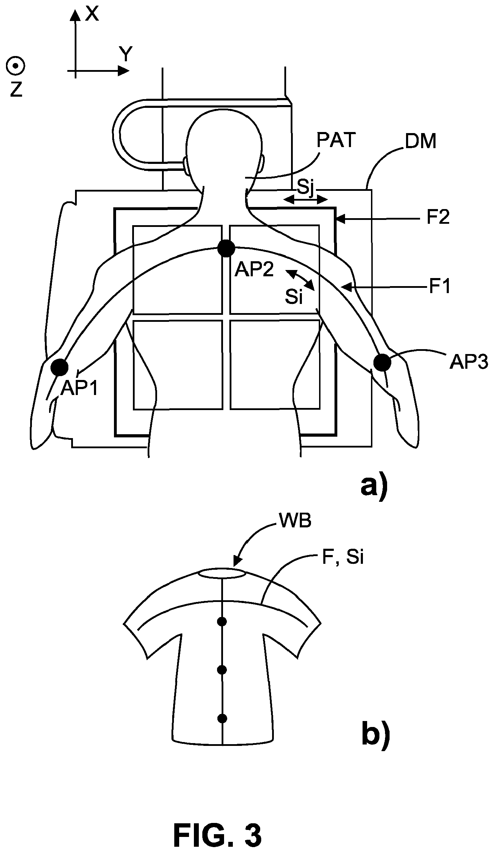

Figure 3a) shows an embodiment of how fiber F1 could be arranged at the patient in respect to the region of interest (in this case the torso or lungs of the patient) that is to be imaged. By choosing suitable anchor points AP1-3, such as on the patient's spine and/or on the patient's elbows for example, a layout of the fiber can be defined that results in the fiber deforming in response to relevant posture changes of the patient. Three such anchor points AP1-3 are shown, but there may be more or less of such anchor points. At least two anchor points are advisable, and these can be defined based on anatomical knowledge and location of the region of interest.

For example, as patient moves their torso, arms, shoulder blades, etc, the fiber F1, suitably arranged as shown, will deform and thus cause tell-tale shape readings that can be correlated to the correct target posture pre-defined by the imaging protocol. Thus, for example, lung region can be imaged with shoulder blades outside the FOV, or at least sufficiently far removed from lung tissue to be imaged to so avoid unwanted overlap in the projection imagery.

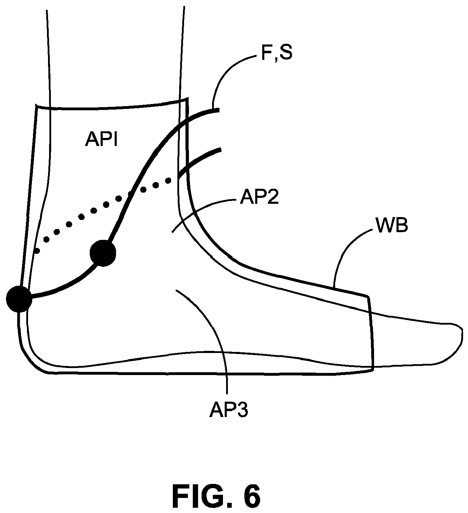

Anchor points on patient's anatomy may include spine, and elbow joints for chest radiograph. The set of anchor points AP1-3 will be predefined for atypical radiography view and preferably bony landmarks with preferable low (such as minimal) relative motion between skin and bone may be selected. For example, this may include regions where bone is subcutaneous with no to minimal intervening soft tissue between the skin and bones. Consideration of suitable anchor points may be such that a set of anatomical landmarks should enable unambiguous determination of the pose of the body part studied. Thus, anchor points are preferably chosen at suitable anatomical landmarks. Selection of such anchor points are based on anatomical knowledge and the type of posture one wishes to monitor. Some typical, non-limiting examples may include tips of spinous processes of vertebrae, olecranon and the two epicondyles for the elbow joint, medial and lateral malleoli and calcaneal tuberosity for the ankle joint, etc (see Figure 6 below).

As also shown in Figure 3, the optional, second set of optical fiber F2 can be arranged at the detector module DM, for example, in a rectangular design running along the edges of the detector module. In general, the fiber may be run to outline FOV of the detector module, not matter its shape, rectangular or not. The second set of optical fibers F2 may be arranged proximal to the patient on the side of the detector module DM facing the patient during imaging as shown. Alternatively, detector module DM may be arranged distal, on the other side of detector module, directed away from the patient (not shown). The sets of shape sensing sensors along the length of the fiber are indicated schematically in Figure 3. Thus, Si and Sj as the two different sets of sensors, one set in the detector fiber F2 and one set in the fiber F1 attached to the patient.

Attachment of fiber F1 to patient PAT may be done in numerous ways, such as by straps (eg, of hook-and-loop fastener type), or by sticky tape or by any other fastening means suitable for coupling to the patient's skin for example. However, direct coupling to skin is not necessarily required as the fiber F1 may be integrated into a wearable WB such as a garment. For example, the fiber may be arranged at a back portion of a clinical gown, shirt, sweater, etc, worn by patient PAT during the imaging. Such as wearable, a garment with integrated fiber, is indicated schematically in Figure 3b). It follows the anchor points AP1-3 layout as defined in Figure 3a), so is configured for Chest X-ray imaging. Depending on the imaging task at hand, garment WB may be embodied instead as a pair of trousers, headwear, etc for imaging ROIs located elsewhere. The garment may include multiple set of fiber, each pre-positioned for different imaging tasks/ROIs so the same garment may be re-used for such different ROIs. Alternatively, the garment includes suitable positioned fabric loops, sleeves or similar attachment means so the garment may be reconfigured, for example by re-threading the fiber F1 accordingly to achieve re-positioning of fiber F1. The garment WB may include preferably a wireless interface to wirelessly transmit the shape readings to interface IN of system SYS for processing into patient posture output data. Bluetooth, WIFI IEEE 802.11 or other communication protocols may be used. However, tethered solutions are not excluded. They may afford less user comfort, but may help reduce signal interference.

Optionally and separately, a further set Sk of sensors, such as a third optical fiber F3 (not shown) may be arranged at the x-ray source XS housing HS so as to allow ascertaining the correct inclination of the x-ray beam, and thus its orientation. The fiber F3 is arranged so that its sensors respond to changes of imaging geometry, such as re-orientations of the x-ray source XS.

This is of particular benefit in free-type imaging setups, such as mobile imagers, where there is no mechanical coupling of detector vs source. In such setups, the central beam direction is not locked to impinge detector's plane at an angle of 90° which is preferable a typical musculoskeletal or mobile chest exams. There may be an unwanted angulation. Use of the third fiber F3 may allow applying the principles described above for tracking X-ray beam direction. For example, the 3rd fiber F3 may have one of its ends attached to a side of tube housing HS, and point into a direction parallel to the central beam. Knowing an offset from the central beam in respect of focal spot FS location, both, the position of the focal point FS and the central-beam direction can be inferred from the shape sensing readings received from this 3rd fiber F3. Such a fiber-based measuring of the focal-point position can also be beneficial for a locked detector tube-head configuration, for example for measuring source image receptor distance (SID). Thus, the principles described herein may be used to measure the imaging geometry. The so determined imaging geometry may be used for example to simulate an "expected" X-ray based on a 3D model of patient PAT.

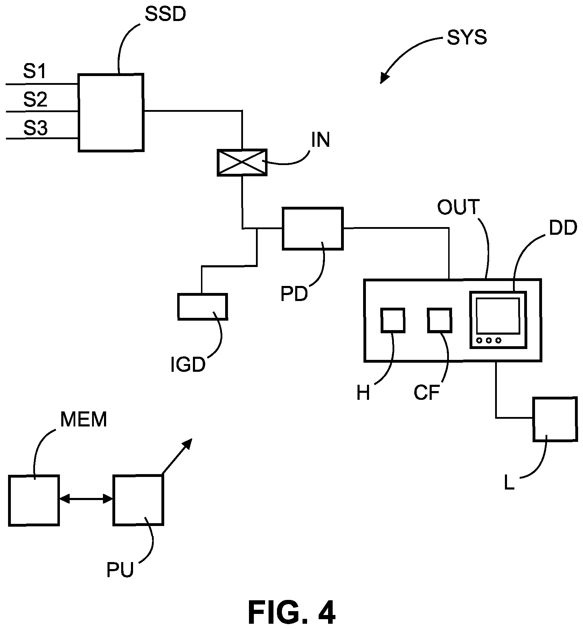

The block diagram of Figure 4, to which reference is now made, represents components of the patient posture tracker/control facilitator system SYS. The system SYS may include one or more input ports/interface IN at with shape sensing measurements/readings of the shape sensing device SSD are received. Input port IN may be arranged for wired or wireless communication as mentioned before.

Preferably the system SYS is of the multi-channel type with multiple fibers providing different sets of readings as per their respective aet of sensors S*i*, S*j*, S*k* arranged at the patient in relation to the region of interest, the detector module DM and at the X-ray source, respectively. The detector fiber F2 and the Source fiber F2 are optional however and a mono-channel setup is also envisaged. That is, in some embodiments it may be sufficient to calibrate the system to work solely on local co-ordinates, thus only collecting measurements from a single or more fibers F1 arranged only at the patient. No fibers F2, F3 at the detector DM or source XS are required. That is, fiber F2 including the second sensor set S*j* arranged at the detector XD is optional, and so is fiber F3 including the third sensor set Sk that may provide measurements in relation to posture changes of the x-ray source XS. However, a multi-channel setup is still preferred as said. This is because having separate sets of sensors in separate fibers arranged respectively at both, the patient's ROI and at the x-ray detector module DM, allows for a more robust and accurate determination of patient posture relative to the x-ray detector, in particular relative to the imaging plane (*X,Y*) which includes the x-ray sensitive pixels.

The fiber F1 at the patient is arranged for example to partly or fully surround or encircle the region of interest which may be an internal anatomic feature, such as one or more of the lungs. More than one fiber may be arranged at the patient, coupled to the skin, to form an imaginary geometrical 2D surface in 3D, at least partly, or possibly completely, enclosing therewithin the region of interest. However, such at least partial enclosing is not necessarily required, and it may be sufficient for fiber F1 to trace out a curve in 3D, passing the ROI as shown in Figure 3. Whatever the geometrical layout of patient fiber F1, it is preferably done to that its sensors can respond to posture changes in the vicinity or surrounding of the region of interest. The region of interest itself may not necessarily move during the posture change, but it may be surrounding by body parts that do, such as shoulder blades or arms, etc that are meant to be kept outside the field of view in lung imaging.

The second fiber F2 at detector module DM, if used, may be arranged to define a plane, which includes, or at least is parallel to the image plane (*X,Y*) of detector pixels.

The shape measurements in relation to patient PAT and at the detector module DM are received at input port and are processed by a posture determiner PD. An optional imaging geometry determiner IGD may process the shape sensing readings Sk received in relation to the x-ray source XS. Operation of the patient's posture determiner PD and imaging geometry determiner IGD may be similar, and the explanations below on operation of posture determiner PD are of equal application to operation of imaging geometry determiner IGD.

Posture determiner PD outputs, based on the received measurements from sensor Si and, optionally Sj are provided as output data at output interface OUT. In the following, a reference to sensor data or sensor input data, sensor feeds, readings, etc, is taken to include reading from the patient sensor Si, and may optionally (but preferably) also include the readings from the second sensor set Sj at the detector module DM.

The output data may be indicative to whether the target posture is achieved, or, if it was achieved before, has been maintained. A logic L may be used to interpret the output data and to issue, for example, a control decision based in the output data. The control decision by logic L may be effected through suitable control interface IF, driver, etc. The logic L is optional, and it may be envisaged to use the output data itself without said logic L, such as to effect displaying the output data on a display device DD, or for tasks other than displaying, such as recording, logging or otherwise storing in a memory, etc.

In preferred embodiments posture determiner PD preferably processes real-time sensor feeds from sensors Si (and optionally sensors Sj), to enable patient posture tracking in real time over a period of time, in particular during x-ray imaging or in a period before imaging. The latter mode/option is to facilitate that the current patient posture is at least within a pre-defined margin of the target posture. For example, logic L may allow commencing imaging only if this is the case. The margin may be configured to reflect measurement error bounds.

Thus, the logic L may in some embodiments issue a binary control signal based on the posture determiner PD's output data. The binary signal may depend on whether or not the output data is indicative of the target posture being maintained or whether it has been achieved. Logic L may be arranged to process the output signal to determine whether the current posture is maintained or is achieved.

The posture determiner PD's output data, or optionally of the output signal produced by the logic L, may be used to accomplish any one or more of a number of tasks. For example, in one embodiment, in a display task, the current posture and/or an indication of its deviation from target posture is indicated in a graphics display on the display device DD, or on a separate display device. The graphics display may include graphical or numerical elements (eg, coordinates, etc), or a combination thereof to visualize current posture and/or deviation.

In some embodiments the output interface OUT includes or is coupled to a control interface IF for controlling imaging operation of imager IA. The control interface IF may couple with control interface circuitry at the imaging apparatus. The logic L may issue a control signal to control in particular switching on/off the x-ray source XS. For example, if the patient target posture is achieved or maintained, imaging is started, resumed, or continued as the case may be. For example, if it is found by logic L that there is sufficiently high enough deviation, imaging is delayed or interrupted. If no such deviation is found, imaging is allowed to start, to continue or resume. The said switching on/off of source XS may include hard switching or grid switching, the letter being preferred. Control by logic L of other imaging tasks, or imaging supportive task, based on posture determiner PD's output data is also envisaged, either in addition to any of the above, or instead. For example, an alert signal may be generated as one option, controlled by logic L based on the output date produced by posture determiner PD. The alert signal may be indicated on the screen DD to inform user, whether or not, the target posture is achieved or maintained. Alternatively, or in addition, the alert signal may to switch a lamp, sound out a sound, etc.

Yet more specifically, one or more haptic actuators H may be controlled by logic L through control signal based on posture determiner PD 's output signal. In particular, the haptic actuator H may be arranged at the patient, for example may be integrated into the fiber F 1 itself. It may be arranged a mechanically actuatable elongated component such as wire, cable, etc that is run alongside the fiber.

The actuator H may be attached independently and separately from fiber F1. For example, haptic indicator H may be arranged as artificial muscle (also known as "muscle-like actuators") that can be controlled to expand and contract in a range of technologies, such as electric field actuation, pneumatic actuation (PAM), thermal actuation, and others still.
logic L may control, based on the output signal provided by the posture determiner, haptic actuator H to encourage patient PAT to correct/modify their current posture, thus urging or guiding the patient, preferably in a real time, and preferably in dynamic manner, to assume the correct target posture. Such actuators H induce forces onto patient PAT to encourage patent to change a posture or remain in a posture. This can be achieved by expansion or contraction, or by varying the twist or stiffness of the muscle-like actuator that is attached to the patient skin. Thus, using the said actuators H in this manner may be understood to mimic haptic cluing guidance usual down by a human user. The setup may thus be used in robotized/autonomous imaging.

Using the above-described feedback options administer by logic L may not be suitable for patients that are not able to respond adequately to such closes, such as infirm, frail patients such as elderly patients or trauma-patients in ICUs, etc. In this case, control tasks envisaged herein for logic L may include controlling, based on output from posture determiner PD, via suitable interfaces, actuators (not shown) that actuate other hardware equipment of the imaging apparatus to facilitate correct posture. For example, the source XS and/or detector DM may be equipped with motorized movement capabilities, controlled by logic L. Alternatively or in addition, patent support PS may be moved via actuators to move patient to thereby achieve correct posture, or at least achieve an improved posture over the current posture.

Some or all of the components of system SYS may be stored on one or more memory devices MEMs. The system SYS may be implemented by one or more processing devices PU as shown in the lower left-hand side of Figure 4. In particular, posture determiner PD and control logic L may be implemented on the same or on different computing systems or devices. Each may be arranged in hardware, in software or in both.

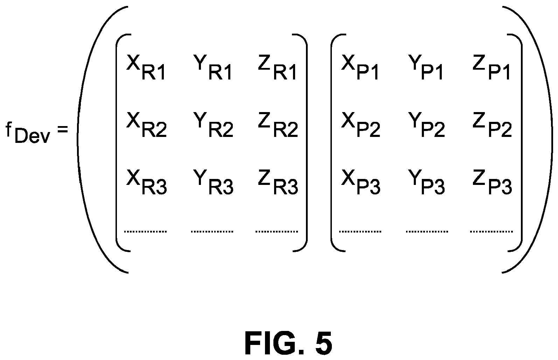

Reference is now made to Figure 5 which shows operation of the patient determiner PD in yet more detail.

As explained above, in the at least two channel embodiment of shape sensing device SSD, separate shape sensing readings are received from sensors Sj at the detector module DM and the second set Si at the patient. Using such a two-channel approach allows for a robust and accurate determination of patient posture versus detector. However, this does not exclude an alternative embodiment, where separate shape sensing devices SSD are used, each with their own sensor sets, and to then later correlate the measurements received therefrom.

In either case, posture determiner PD may implement a function *f_{DEV}* that measures the current posture, or preferably, its deviation from the target posture. The values of function *f_{DEV}* are preferably scalar values suitably calibrated, so that one value, a target value for example a zero value, indicates that target posture is achieved. Values larger or smaller than the target value indicate there is deviation, and the value of function *f_{DEV}* varies with amount of deviation.

The target posture, preferably in combination with detector DM plane may be considered a reference model. The deviation function *f_{Dev}* compares patient curvature points vector with this reference model to detect the positional deviation.

Shape measurements (*X,Y,Z)_{Rj}* represent reference frame measurements for example received from the detector sensor set Sj (fiber F2), whilst (*X,Y,Z)ₚₖ* represents measurements received from the first sensor set Si (fiber F1) arranged at patient PAT. As illustrated in Figure 5, the two sets of measurements (*X,Y,Z)_{Rj}* (*X,Y,Z)ₚₖ* may be stored as matrices and processed using matrix operations for effect storing and processing. The shape measurements may include 3D curvature points, stress/strain measurements or any other quantities related to shape of the respective fiber or system of fibers is used collectively, such as patient F1, detector fiber F2 and optionally source fiber F3.

The two sets of shape measurements may be combined suitably by function *f_{DEV}* to compute the deviation value.

For example, a set reference measurements is obtained [(*X,Y,Z)_{Rj}*, (*X,Y,Z)pₖ*]^{R} and this compared with current measurements [(*X,Y,Z)_{Rj}*, (*X,Y,Z)_{Pk}*]^{C}*.* This may then be processed by distance function *D*([(*X,Y,Z)_{Rj},* (*X,Y*,*Z)_{Pk}*]^{R}*,* [(*X,Y,Z)_{Rj}*, (*X*,*Y*,*Z)ₚₖ*]^{C}) to arrive at deviation value *f_{DEV}.* For example, the Euclidean component-wise difference may be formed (*of* [(*X,Y,Z)_{Rj},* (*X,Y,Z)_{Pk}*]^{R} and [(*X,Y,Z)_{Rj}*, (*X*,*Y,Z)_{Pk}*]^{C}, preferably squared. Higher moments may be used instead, or any other distance function may be used, as required.

The measurements (*X,Y,Z)_{Rj}* collectable at detector module are optional and the above formulae may be used based on patient-side measurement (*X*,*Y,Z)_{Pk}* only, for example if there is no motion expected of the detector DM.

Similar computations as described above based on *f_{DEV}* may be done by the imaging geometry determiner IGD. Imaging geometry determiner IGD uses measurements collected by sensor set Sk (such as fiber F3) at the X-ray source. This can be combined with measurements collected by set Sj (such as fiber F2) at the detector. The Imaging geometry determiner IGD may thus determine deviation from a target imaging geometry pre-defined for the imaging task at hand. Imaging geometry determination may be done jointly with patient posture determination. Thus, inadvertent imaging geometry changes or incorrect imaging geometry settings causes by inexperience or fatigued staff may be detected. Logic L may thus prevent imaging by examining *f_{DEV}* values in relation to both, patient and imaging geometry, and allow/recommend imaging only if both are on target.

In a calibration phase prior imaging, patient is asked under the guidance of a medical practitioner for example, to assume the correct target patient posture in front of the detector module DM. Whilst remaining in this target position shape, measurements are registered, and these are stored as reference values representative of the target posture. This can be a one-off operation for a given patient, and could be reused for other patients, or indeed for the same patient for later imaging sessions. Preferably, calibration is done in a dedicated calibration phase for each patient individually. This individualized "tailored" approach, whilst more time consuming, may be preferred as the target values may deviate slightly for each patient given different bio-characteristics, such as sex, height, BMI, etc. In a semi-tailored approach, for a population of patients with different bio-characteristics, respective target values may be stored in a database, stratified according to imaging task/target posture type. Then, at a later stage, for a given patient, the target value matching bio-characteristics of a given patient may be retrieved and applied by function *f_{DEV}.*

In some embodiments, a setup phase may include "calculating the shape" of the detector plane of detector DM. This may be done by recording readings from fiber F2 once this has been put in place, for example by placing fiber F2 along the circumference of detector plane with its four corner points (see again Figure 3a). In the same manner sensor readings for patient PAT are received from fiber F1 when patient is in the correct posture. This may be referred to as "calculating patent shape". In this way, the reference model (in this case optionally including the detector readings from fiber F2 may be established.

During imaging, current shape measurements are received in a stream at a certain sampling time. The shape measurements will vary with patient posture changes, for example caused by involuntary movements, such as cause by coughing etc. Function *f_{DEV}* may thus be configured to measure difference between the target shape measurements and the current measurements to establish the deviation value.

In one embodiment, a Hamiltonian quaternion setup may be used to compute *f_{DEV}.* Specifically, let *(x1,y1)* be the plane representing the reference frame as defined by the detector fiber F2, and *(x2,y2)* the plane representing the patient frame as defined by patient fiber F1. Let *P1* denote a center of the reference frame at detector, and *P2* the normal vectors to the (*x1,y1*) and (*x2,y2*) plane, respectively. Quaternion *Q*(*P1, P2*) then represents the relationship between *P1* and *P2* vectors, whereas one component of the quaternion ( sometime referred to as "*W*") represents an angular relationship. This angular relationship can be projected on to other planes to find out the components *θ*, *Φ*, which, along with r (distance between patient and detector) defines the complete set of polar co-ordinates of the patient frame.

A similar setup as discussed above at Figure 5 may be used for the imaging geometry determiner IGT, which processes measurement collected by the optional third set *S*ₖ of sensors (*X*,*Y,Z)_{Tj}* at source XS.

With continued reference to the controlling of the haptic H actuator, in order to guide patient to achieve target posture, the patient fiber F1 or the detector fiber F2, may be arranged one or both along 3 spatial directions. The above defined function may then allow not only computing whether or not there is deviation, but to localize the deviation in 3D. Thus, the deviation may be mapped as respective spatial components onto the three spatial axes. The haptic actuator may also be arranged in a 3D layout, and the respective spatial components. The respective spatial components may thus encode or indicate along which spatial action there needs to corrective user motion, and by how much. The logic L may use respective spatial components to produce the localized control signal to drive actuator H so as to indicate haptically a directive action in 3D to so guide user for corrective motion to achieve target posture.

For example, one may conceptually divide each of the spatial axes into a positive and negative part to so define quadrants. For example, "patient left" is coded as negative, and "patient right" is coded positive (-| +). Assuming the X component of the correction vector representing left to right horizontal position, activation of specific haptic sensor is chosen based on the sign of the X component. The Haptic duration may be based on a magnitude of X for example. The same can be done for any of the other two axes. Again, a Hamiltonian quaternion setup may be used to formulate localization of deviation and translating this into a suitable corrective vector, which may be sounded-out, displayed or otherwise brought to user and/or patient attention.

Figure 6 illustrates an alternative use of the above-described principles in an imaging session other than for chest x-ray, in particular, for orthopedic applications, for example in respect of the human foot. The suitable anchor points AP1-3 at the foot may for example include any two or all of i) medium malleolus, ii) lateral malleolus, and iii) calcaneal tuberosity, as required. As can be seen, fiber F1, F with set of sensors S may be integrated into footwear, such as a sock WB for example. Fibers F1 F, and hence sensors S may be integrated in such a manner that fiber F, F1 (and hence its sensor set S) passes through the anchor points AP1-3, when the sock is worn by patient PAT during imaging session.

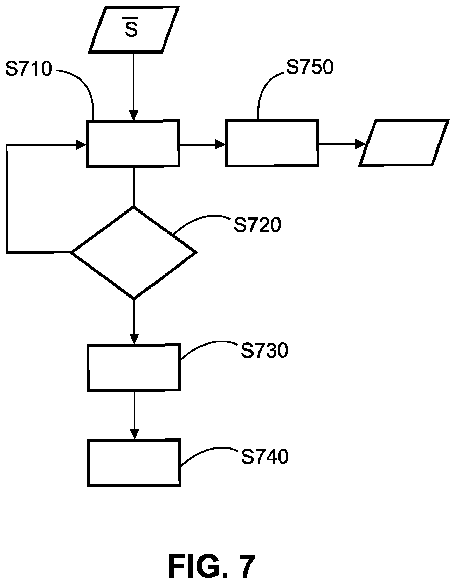

Reference is now made to the flow chart in Figure 7 which shows steps of a computer implemented method for implementing the above system SYS. However, it will be understood that the below described steps are not necessarily tied to the architecture shown above.

The method is one for establishing, preferably in 3D or at least in 2D, a current patient posture and/or whether this deviates from a desired target posture. The method is preferably applied during imaging of a patient in an imaging session, or before imaging is t take place. The imaging may be done by an imaging apparatus, preferably, but not necessarily, of the x-ray type. Projection X-ray imaging such as in chest X-ray, orthopedic imaging, mammography, etc is envisaged herein in embodiments, and so are CT imaging, MRI or PET imaging.

At step S710, one or more sets of shape sensing measurements are received. Optical shape sensing is preferred herein. One such set of measurements may originate from a set of sensors. The sensor may be included or are part of a deformable probe of the shape sensing device, such as an optical fiber. The sensors/fiber/probe may be arranged at the patient in relation to the region of interest. The measurements are collected in a manner so that they correlate to posture changes in respect of the region of interest and/or to surrounding body parts or anatomies.

The shape sensing measurements are processed at step S720 to compute output data, such as a value that is indicative of whether there is any deviation of a current posture in respect of a predetermined target posture, or where the value if indicative of a current pose, as required.

At step S730 an output is provided including the output data.

At step S740 an output signal is established, based on the output data. The output signal may be used for any one or more of a number of processing or control tasks. The method may thus further comprise step S760 of controlling or performing such a control task based on this signal. Step S740 may be implemented by a logic, such as in hardware, software or both.

For example, one control task includes control of image acquisition. For example, the x-ray source is controlled, based on the control signal. For example, the x-ray source is disabled or switched off so that no x-ray beam leaves the source's egress window in case there is such deviation. Imaging is resumed, that is the x-ray beam is allowed to egress the egress window, once the target posture is achieved or is within a pre-defined margin thereof. Alternatively or in addition, image acquisition is only allowed to commence once target posture is achieved. Thus, this step may act as a delay switch to delay imaging until target posture is achieved. The control operation during imaging may be ongoing in a monitoring operation to interrupt imaging acquisition and/or to issue an alert signal to use, once posture is no longer on target.

The controlling S760 may relate to any one or more components of the imaging apparatus, such as actuators for moving patent table, detector, source, etc.

Preferably, the shape measurements received at step S710 comprises a second set of measurements collected from a second set of sensors arranged at the detector module. The sensor may be part or included in a separate fiber (separate from the patient fiber), which is integrated or coupled to/at or in the x-ray detector module.

Optionally, but less preferred, the second set of sensors is arranged elsewhere in the examination room or at the imaging apparatus, in a pre-defined, know spatial relationship to the x-ray detector module DM. Using such an optional second set of sensors at the detector side allow for a more robust determination of patient posture relative to the detector. In addition, because both streams of measurements from the two sets of sensors are accounted for, any changes in posture of the X-ray detector (inadvertent or not) can be picked up and feed back to steps S740, S760.

At an optional step S750, an imaging geometry is determined. In particular, an inclination of the x-ray beam may be determined or an SID, as required. This can be done by arranging a separate, third set of shape sensing sensor/fiber at the x-ray source as explained above. This step is similar to step S740 above, and a value is computed that represents a deviation between current imaging geometry from target imaging geometry, such as current beam inclination from target inclination. The control task may include starting or resuming imaging, only when the target imaging geometry is set. Thus, imaging is so controlled that both, patient posture and imaging geometry are on target.

An order of steps S750 and S740 is interchangeable.

The method is preferable implemented in real time and in a dynamic manner at a suitable sample rate to operate on a stream of shape measurements as received at step S710 from one or more of the set of sensors, as required.

For present purposes, the above-described principles may also be used for tracking breathing cycles, as the motion of chest in the breathing cycles may be understood as a special case of patent posture in respect to the chest. For example, for chest PA exams, full inspiration is important for diagnostic quality. By tracking the change of curvature of a fiber F1 attached to the patient's chest over time allows for inferring the current inspiration status. For example, fiber F1 may be strapped over the anterior part of the thorax. In this way, user may check whether full inspiration is achieved for finding the best moment to trigger X-ray acquisition. Inspiration tracking can also be calibrated prior to the exam by asking patient to fully inhale / exhale and recording the corresponding curvature.

The method can be used for issuing alerts for patient movement, for example to assist in acquisition timing. For example, after having achieved the target posture, for example under instructions from user, at least parts of the above method may be used in monitoring for possible movements and hence deviation from target posture, for example after user left the acquisition room and about to request acquisition. By defining suitable margin on the maximally allowed deviation und thus movement, the controlling may include suppressing a requested acquisition, in case the deviation exceeds safety margin.

The controlling step of the method may include, in addition or instead, triggering acquisition, if target posture is achieved. This may be referred to as an "auto-acquisition" mode.

When the system is implemented on performant computing equipment such as those using parallel or multicore processors, the auto-acquisition mode may be used to pick up the current posture whilst monitoring the stream/feed shape measurements even in volatile imaging conditions with fast, jerky and frequent motion, such as when imaging children or pets.

The components of system SYS may be implemented as one or more software modules, run on one or more general-purpose processing units PU such as a workstation associated with the imager IA, or on a server computer associated with a group of imagers.

Alternatively, some or all components of the system SYS may be arranged in hardware such as a suitably programmed microcontroller or microprocessor, such an FPGA (field-programmable-gate-array) or as a hardwired IC chip, an application specific integrated circuitry (ASIC), integrated into the imager IA. In a further embodiment still, the system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc. For example, posture determiner PD and logic L may run on different computing devices PU or on the same computing device PU.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.
According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

## Claims

1. System (SYS) for facilitating medical imaging of a patient by a medical imaging apparatus (IA), comprising:
an input interface (IN) for receiving measurements collected by shape sensing sensors (Sⱼ) of an optical shape sensing device (SSD), the shape sensing sensors (Sⱼ) arrangeable relative to the patient's body, wherein the measurements are representative of a current posture of the patient's body; and
a posture determiner (PD) configured to compute, based on the measurements, output data representative of whether the patient's body is in a predefined target posture.

2. System of claim 1, comprising an output interface (OUT) for providing the output data, the output data including and one or more of i) an indication for the said current posture, ii) an indication on when the patient's body is determined to be in the predefined target posture, and iii) indication on when there is a deviation between the said predefined target posture and the current target posture.

3. System of claim 2, wherein output interface (OU) includes any or more of: a display device (DD), a haptic actuator (H).

4. System of claim 1, 2 or 3, comprising a logic (L) configured to recommend to a user to initiate imaging, or the logic (L) to automatically initiate the imaging, if the output data is indicative of the patient's body being determined to be in the predefined target posture.

5. System of any one of the previous claims, wherein measurements include measurements collected by further shape sensing sensors (Sₖ) arrangeable in a predefined spatial relationship to a detector module (DM) of the medica imaging apparatus.

6. System of claim 5, wherein the further shape sensing sensors (Sₖ) are arrangeable in a layout that defines a reference plane.

7. System of any one of the previous claims, wherein the said further shape sensing sensors are arrangeable in, at or on the detector module.

8. System of any one of the previous claims, wherein the shape sensing sensors are arrangeable on the patient's body.

9. System of any one of the previous claims, the shape sensing sensors are includable or couplable in or to a wearable (WB).

10. System of any one of the previous claims, wherein the measurements include yet further measurements collected by yet further shape sensing sensors (Sl) arrangeable at the imaging apparatus, wherein the system further includes an imaging geometry determiner (IGD), configured to determined based on the further measurements, a current imaging geometry of the imaging apparatus.

11. An imaging arrangement (AR), including a medical imaging apparatus, and shape sensing sensors arranged at the imaging apparatus, and supplying the shape measurements to a system as per any one of the previous claims.

12. The arrangement of claim 11, the imaging apparatus (IA) having a detector module, the shape sensing sensors arranged on or at the detector module.

13. Computer-implemented method for facilitating medical imaging of a patient (PAT) by a medical imaging apparatus, comprising:
receiving (S710) measurements collected by shape sensing sensors (Sⱼ) of an optical shape sensing device (SSD), the shape sensing sensors (Sⱼ) arrangeable relative to the patient's body, wherein the measurements are representative of a current posture of the patient's body; and
computing (S720), based on the measurements, output data representative of whether the patient's body is in a predefined target posture.

14. A computer program element, which, when being executed by at least one processing unit, is adapted to cause the processing unit (PU) to perform the method as per claim 13.

15. At least one computer readable medium (MEM) having stored thereon the program element of claim 14.
